# EUROPEAN PATENT SPECIFICATION

(11) **EP 3 215 082 B1**
(45) Date of publication and mention of the grant of the patent: **14.04.2021**
(21) Application number: 15790145.5
(22) Date of filing: 05.11.2015
(51) Int. Cl.: A61F 13/15, B29C 55/02, B29C 55/06, D06J 1/00, D06C 3/00

(54) **SELECTIVE MACHINE-DIRECTIONAL STRETCHING OF A PRECOMBINED ELASTIC WEB OF VARYING WEB WIDTH BY SPEED DIFFERENTIAL STRETCHING**
SELEKTIVE MASCHINENDIREKTIONALE STRECKUNG EINES VORKOMBINIERTEN ELASTISCHEN GEWEBES MIT VARIIERENDER GEWEBEBREITE DURCH DREHZAHLDIFFERENTIALSTRECKUNG
ÉTIREMENT SÉLECTIF DANS LA DIRECTION DE LA MACHINE D'UNE BANDE ÉLASTIQUE PRÉCOMBINÉE DE LARGEUR DE BANDE VARIABLE PAR ÉTIREMENT PAR DIFFÉRENTIEL DE VITESSE

(30) Priority: 05.11.2014 GB 201419705
(43) Date of publication of application: 13.09.2017
(73) Proprietor: Concepts For Success (C4S), 53881 Euskirchen (DE)
(72) Inventor: SCHMITZ, Christoph, 53881 Euskirchen (DE)
(74) Representative: Plischke, Manfred
(86) International application number: PCT/EP2015/075774
(87) International publication number: WO 2016/071429

(56) References cited:
- WO-A1-92/15444
- WO-A1-2005/065932
- US-A- 4 654 180
- US-A1- 2006 083 900
- US-A1- 2014 041 786

## Description

### Field of the invention

The present invention relates to a process for selectively stretching sections of precombined web materials which comprise fibrous material as well as elastic material and which exhibit a varying web width by speed differential stretching. Such materials may be suitably applied in disposable articles, such as hygiene articles like baby diapers, adult incontinence articles and the like. The present invention also includes apparatus for manufacturing such stretched web materials.

### Background

It is well known to impart extensibility to a web by mechanically activating the web between two intermeshing rib or corrugation structures.

In US4834741 (Sabee) a disposable garment is described, where one or more elastic elements are affixed in a relaxed condition between a topsheet and backsheet. The web is then stretched, and simultaneously the web in the area of elastic element attachment is drawn to permanently extend the fibres. Upon relaxation of the stretched elastic element, shirrs or gathers are formed in the web fibres that permit subsequent stretching of the elastic element when the garment is worn. The apparatus includes rolls for pre-corrugating one garment sheet and for simultaneously stretching the elastic element and drawing the web in the region of elastic element attachment. Alternately, the entire web may be drawn, rather than just in the region of elastic element attachment. The corrugated rolls may have meshing corrugations aligned substantially parallel to the machine directions - so as to impart cross-directional extensibility - or substantially cross-directionally - so as to impart machine - directionally extensibility.

In WO1992/015446 it is disclosed to incrementally stretch a "zero strain" stretch laminate material by passing it through an incremental stretching system, such as the nip formed between a pair of meshing corrugated rolls which have an axis of rotation substantially perpendicular to the direction of web travel.

It is also known to stretch a web material by differential speed rolls and to combine such a differentially stretched elastic web material with another, essentially inelastic web material to impart shirring of the inelastic web, allowing elastic expansion and retraction up to the break point of the inelastic web, see e.g. US8591625, or JP2001346825. The maximum extension of the web is length of the inelastic web prior to the combination, and the maximum shirring is determined by the stretch of the elastic web prior to the combination.

In US2003/0067091(A1), a process for enhancing breathability properties of webs comprising films with mineral fillers is described, wherein the web is stretched in the longitudinal direction of the same, in two successive steps, and an embossing is carried out, at least in certain sectors of the surface, between the two steps which effect the stretching in the longitudinal direction.

In WO2008112517A1 a web stretching method is described, wherein a deformation zone is formed between an activation belt and an activation roll both with inter-engaging teeth and grooves at a controlled depth of engagement capable of increasing linearly over the deformation zone.

However, prior art fails to provide a solution for extending machine directionally spaced apart portions of a web comprising an elastic and an essentially inelastic sublayer and exhibiting a repeating pattern of effective web width, by stretching web portions selectively between rolls operating at a speed differential, thereby imparting machine directional elasticity to narrower regions whilst leaving other regions essentially inelastic.

### Summary

The present invention is a method for selectively machine directionally stretching a predetermined section of a pre-combined web material. In a first execution, the method comprises the steps of
a) providing an essentially continuous pre-combined web exhibiting an x-direction aligned with the machine direction, a width corresponding to the cross machine direction and a thickness perpendicular thereto at an infeed speed. The web comprises
   a1) at least one essentially non-elastic fibrous sub-layer and
   a2) at least one elastic sub-layer,
      the sub-layers being connected to each other,
   and the web further comprises in a repeating sequence a first region exhibiting a narrower effective material width than a second region, wherein the first regions defines a first pitch length, and the second region defines a second pitch length, both defining, optionally with further repeating regions or transition regions, a repeating infeed overall pitch length;
b) providing a selective stretching tool comprising a first and a second roll, defining a stretching zone along the web path between them;
c*) operating the second roll at a preset and essentially constant speed essentially corresponding to the outfeed speed;
d*) operating the first roll at a predetermined speed, which is
   d1 *) either constant at a speed lower than the second roll speed
   or d2*) oscillating between
      d2i*) a speed lower than the second roll speed and a speed equal to the second roll speed or
      d2ii*) a lower speed and a higher speed, such that the integral over time of the speed profile of the first roll over a time increment corresponding to a pitch length is smaller than the integral over time of the speed profile of the second roll,
         whereby the speed differential between the first and the second roll extends the first region of the web to a greater extent than the second region, thereby increasing the first region pitch length as well as the overall pitch length for the outfeed compared to the infeed. Preferably, a buffering step may be provided in the web path after the second roll.

In an alternative execution, the function of the first and second roll may be reversed, such that as a step c**) the first roll may be operated at a preset and essentially constant first roll speed which essentially corresponds to the infeed speed of the web, and d**) the second roll is operated at a predetermined second roll speed, which is either d1**) constant at a speed lower than the first roll speed, or d2**) is oscillating between d2i**) a speed higher than the first roll speed and a speed equal to said first roll speed, or between d2ii**) a lower speed and a higher speed, such that the integral over time of the speed profile of the second roll over a time increment corresponding to a pitch length is smaller than the integral over time of the speed profile of the first roll. In this execution, a buffering step is preferably included in the web path prior to reaching the first roll.

The outfeed overall pitch lengths preferably exceeds the infeed overall pitch length by less than 20%, preferably less than 10% but more than 3%, preferably 5%, based on the infeed overall pitch length. The first pitch length is preferably increased from the infeed to the outfeed by less than 50%, preferably less than 30%, more preferably less than 20%, based on the first infeed pitch length.

In a particular execution, step d*) or d**), respectively, is executed at a first roll speed, which is constant at a speed lower than the outfeed speed, wherein the pitch length after stretching is less than 100% of the stretch zone.

In another particular execution, step d*) or d**), respectively, is executed at first roll speed, which is oscillating between a lower speed and a higher speed, such that the integral of the speed profile of the first roll over a time increment corresponding to a pitch length is smaller than the integral over time of the speed profile of the second roll, and wherein the stretch zone length is less than 50%, preferably less than 20% of the infeed pitch length, based on the infeed pitch length.

The present invention also relates to a method of forming a disposable article, which comprises a front portion and a rear region connected by a crotch region, thereby defining the length direction of the article. The article further comprises a centre strip positioned in the front, crotch, and rear portion. The method of forming the article may comprise the steps of
- positioning an essentially inelastic web material for forming the centre strip of the article such that the machine direction of the method is parallel to the length direction of the article, such that the inelastic web material exhibits two longitudinally extending margins;
- selectively machine directionally stretching at least one predetermined section of a pre-combined web material according to any of the preceding claims;
- creating two web stripes by separating a pre-combined web material selectively machine directionally stretched in at least one predetermined section along an essentially longitudinally oriented separation line;
- positioning and connecting a first and a second of the selectively stretched materials adjacently to the lateral margins of the centre strip whilst the stretched material are in a stretched state;
- adding absorbent member;
- adding side closure elements preferably selected from the group consisting of melt fusion bonded side seams, adhesively bonded side seams, adhesive closure tapes, mechanically engaging closure tabs.

### Brief description of the Figures

Fig. 1A and B show schematically the method and the respective equipment according to the present invention.
Fig. 2A, B, and C depicts schematically the webs according to the present invention.
Fig. 3 depicts schematically roll speed profiles over time according to the present invention. Same numerals depict same or equivalent elements or features.

### Detailed Description

The present invention is directed towards stretchable or extensible web materials. Such materials may very suitably but without any limitation be employed for the manufacturing of hygienic articles, such as disposable and more particular for disposable absorbent articles, which can be produced on automated high speed production lines, which may be operated at web speeds of more than 300 m/sec or even more than 500 m/sec, such as throw-away underwear, baby diapers, or feminine hygiene or adult incontinence products, but also to durable apparel such as girdles, panties, tights, and so on.

Such articles should generally fit to the body of a wearer. Often fit during use is enhanced by the use of elastic materials, such as employed as leg elastics, elasticized side panels, or even fully elasticized articles. Therein, the elastic stretch material may be essentially linear, such as elastic threads, or two-dimensional, thereby exhibiting a unidirectional elasticity or a bi-directional one.

Thus, elastic material may be in an essentially relaxed state (i.e. no retracting force) prior to being applied to a wearer and in an extended state during donning and/or use. For the latter, it is often desired not to have strong retractive forces so as to avoid irritation of the skin of a wearer, but to rather have small forces allowing the material to adapt to the body contours. Typically, elastic materials are used in combination with other webs, such as film and/or nonwoven materials, as well known in the art. The term "web" relates to any material which is essentially endless or continuous in one direction (generally denoted as "x-direction" corresponding to the "machine direction"). Webs are often, but not necessarily, stored, supplied or used in roll form and thusly also sometimes denoted "roll goods". Whilst these are then not "endless" in the strict sense of the word, their extension in this x-direction is significantly larger than in any other direction. By combining consecutive rolls or other batches, ("splicing") such webs can be considered "endless" for all practical purposes. Webs may be transported in a "batch" form, such as when a roll thereof is shipped, or they may follow a "web path", such as when the webs are unwound from a roll, as described hereinafter.

Often, but not necessarily, webs have an essentially uniform thickness (herein denoted as "z-direction", and a constant or varying width (herein denoted as "y-direction") along the x-directional length. Along the width direction, webs may have discontinuities, such as slits or apertures, such that the overall width may be constant, but the effective width may vary and correspond to the overall width in parts of the web, which do not exhibit such discontinuities, and may be smaller elsewhere. Webs may be of essentially uniform composition, they can be mixtures of materials, they can be composites of materials such as being layered (different materials arranged in a juxtaposed position in the z-direction) and/or can comprise stripes of different materials or materials having different or varying properties (i.e. arranged in a juxtaposed position in the y-direction). Typical examples for webs are - without implying any limitation - plastic films or foils, textiles, nonwovens, nets, scrims, paper, or cartons. A web suitable for the present invention comprises at least two sublayers, wherein at least one thereof is a fibrous essentially inelastic material, and at least one is an elastic material. A fibrous sublayer material may be a preformed web structure itself, such as a nonwoven material, optionally consisting of sub-strata itself or may be formed in-situ, e.g. when such fibres are deposited just after spinning or meltblowing onto another web, such as an elastic layer as described herein below. Sublayers may be bonded or pre-bonded by any conventional technique, such as by heat- or melt-bonding, which may be created through compression and/or application of pressure, heat, ultrasonic, or heating energy, cohesion, adhesion, such as by glue or adhesive application. Fibrous materials can be formed by many processes including - without limitation - meltblowing, spunbonding, spunmelting, solvent spinning, electro-spinning, carding, film fibrillation, melt-film fibrillation, air-laying, dry-laying, wet laying with staple fibers, and combinations of these processes as known in the art. Such a fibrous structure may comprise short staple fibres and/or essentially endless spun fibres and/or meltblown fibres of thermoplastic materials as well known in the art, or even certain amounts of short natural fibres such as cellulosic fibres.

The fibrous material of a sublayer may be bonded by any conventional means, such as thermal bonding by thermal compression over the full surface or at bond points or other bonding patterns, or by bonding contacting portions of fibres to each other, such as achieved by through air drying.

"Essentially inelastic" refers to materials, which withstand normal usage conditions without significant deformation. Such a sublayer material will exhibit a deformation under load such that at least for low forces or loads, the fibres will re-arrange without substantially changing the overall shape, resp. length in the stress direction.

At least one other sublayer exhibits elastic properties, i.e. retracts after stretching forces are removed. Elastic materials may be provided "as such" such as in the form of elastic strands, elastic films, elastic webs or sublayers as described in the above, or nets or the like at least comprising, if not essentially consisting of elastic materials, such as - without limitation natural or synthetic rubber, or thermoplastic elastomers like polyolefinic elastomers, such as known as Vistamaxx™.

A web suitable for the present invention comprises at least two sublayers but may include more than two sublayers, such as when an elastic film or fibrous meltblown layer of elastic material is sandwiched between two inelastic webs, such as spunbonded webs. Such a web may be bonded thermally or by applying adhesive or glues between the sublayers, as a full further sublayer, or only partially covering one or both of the connecting surfaces of the sublayers, such as when being applied in stripes or dots or other patterns.

The at least two sublayers are connected to each other to form the web. To this end, such a web may be formed by positioning these at least two sublayers in a contacting relation and connecting these by suitable means. In a first approach such a connecting may be executed as a continuous adhesive bonding, i.e. an adhesive layer is positioned throughout the contact surfaces or a partial adhesive bonding, i.e. an adhesive is positioned to a portion of the otherwise contacting surfaces, such as in stripes, or lines, or dots. The connecting may also be executed by thermal bonding, such as when such sublayers of appropriate composition are positioned relative to each other and at least a portion of at least one of the compounds is molten so as to be able to form a thermal melt connection with the other sublayer, optionally by compressing the sublayers together, preferably in the form of a bond point pattern, or discrete lines. The sublayers may also be brought into contact in a state that allows bonding without adding further heat. This may be achieved for example, when fibres of an essentially inelastic compound are laid onto an elastic layer, e.g. an elastic film or an elastic fibrous web, whilst either of the compounds is still in a bondable state, such as when still being hot respectively fully or partially molten or tacky.

All these options are well known in the art, and require no further explanation. In a preferred execution, the sublayers are bonded homogeneously across the full surface. It should be understood that in case of fibrous webs, contact across the full surface is meant to encompass that the surface is effectively formed as an enveloping line around the web, enveloping the most outwardly portions of fibres, which the form the contact to other surfaces. Thus, in case of two fibrous webs being connected over their full surface, it refers to multiple bonding sites or dots being essentially homogeneously spread over the enveloping line. It is preferable that the bonding of the sublayers is achieved without the use of additional adhesive materials. Often, such materials provide extensibility in one direction, e.g. in cross direction (depending on fibre orientation in the fibrous sublayer) without needing any further treatment.

For web materials suitable for the present invention, the stress-strain properties will initially be dominated by the properties of the inelastic material:
For very low forces and extension values, the fibres of the sublayer(s) will dislocate without impacting any of the bonding and the material will retain its original shape. This "Hook's deformation region", however, is very limited, and nonwoven materials will already after extensions beyond 10% or even 5% (i.e. to a total length of 110% resp. 105%) exhibit a "permanent set", i.e. after releasing the stress the material will not recover to its original length.

Once the extension is increased up to moderate extension values, the stress-strain-curve will exhibit an almost linear increase due to starting to break some of the bonding within the fibrous structure combined with extending the elastic material. Upon releasing the force, the elastic forces will retract some of the length. It should also be noted that elastic materials typically exhibit a hysteresis when being stretched in cycles, wherein for the first load cycle the stress-strain behaviour will be distinct from the following cycles, which preferably do not show major deviations from each other. These principles are well known to a person skilled in the art but also explained in more detail in e.g. WO97/20091,

The present invention is particularly suitable for operating in relatively low ranges of extension. Whilst elastic materials may readily withstand extensions towards more than a multiple of their original length (or extension towards 300 or 400% or even more), the present invention is particularly useful when extensions are more than about 5% or 10%, but also less than about 50%, more preferably less than about 20% of the original length (i.e. to 105% or 110% respectively 150% or 120%).

In this range, the web material will exhibit a certain amount of permanent set, i.e. it will after a first extension not fully regain its original shape, but this difference will not differ significantly for multiple extension cycles.

However, the present is also relating to web materials as described in the above as being suitable, that have been submitted to a mechanical activating step, as described in the above referenced US4834741 or WO1992/015446. Also co-pending application GB 1419704.1 (unpublished) describes a suitable method of selectively stretching a pre-combined elastic web by corrugating. Thus when employing such webs in the context of the present invention, the previously activated webs or web-portions may be elongated to the degree of stretch as imparted by the mechanical activation, and then further and beyond this by the process described herein.

In a particular execution, the web material for being treated according to the present invention may comprise apertures, such as are known in the art to make films gas permeable, and such apertures may change size and shape during the present treatment. In a different aspect, the present treatment or the mechanical activation step described just hereinabove may impart gas permeability in the web, such as by creating apertures in the web by selectively tearing the web material, such as at melt fusion bond points, or when a film as a sublayer comprises particulate filler material that creates micropores upon being stretched. The present invention is thusly directed towards a process and an equipment suitable for executing such a process of modifying such suitable webs comprising at least an essentially inelastic fibrous and at least an elastic sublayer such that they are machine-directionally elastically extensible in sections of the web as being machine directionally spaced apart from sections as being essentially in-elastic in machine direction.

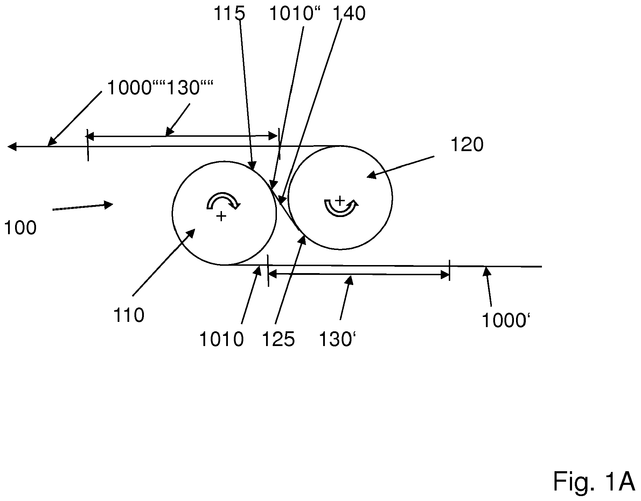

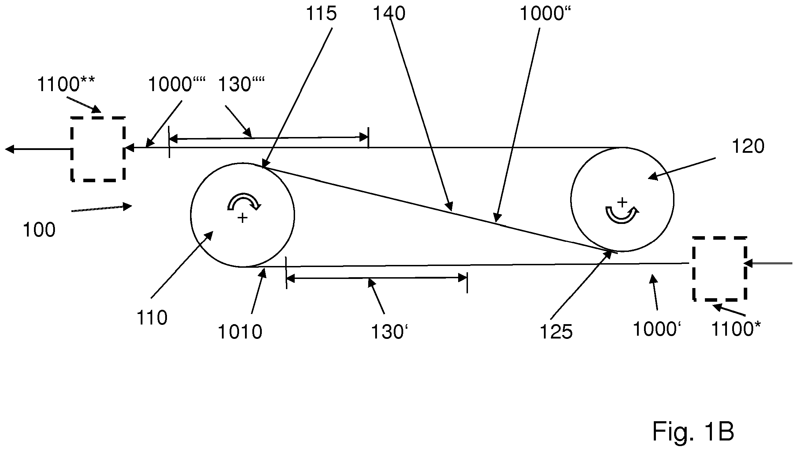

The process is now explained by referring to Fig. 1, which, however, should not be seen as a limitation to the embodiments depicted therein.

A web material as described in the above is provided to a stretching unit 100 from a web supply (not shown). The web 1000 travels along a web path 1010, wherein 1000' denotes the web infeed and 1000"" the web outfeed, as will be discussed herein below. The web exhibits a varying width along its length, respectively machine direction, thereby defining a certain shape or pattern repeating along the web with a pitch length corresponding to the length of an article or to a precursor or another element for being combined with an article or another precursor material for forming such an article downstream of the stretching unit 100.

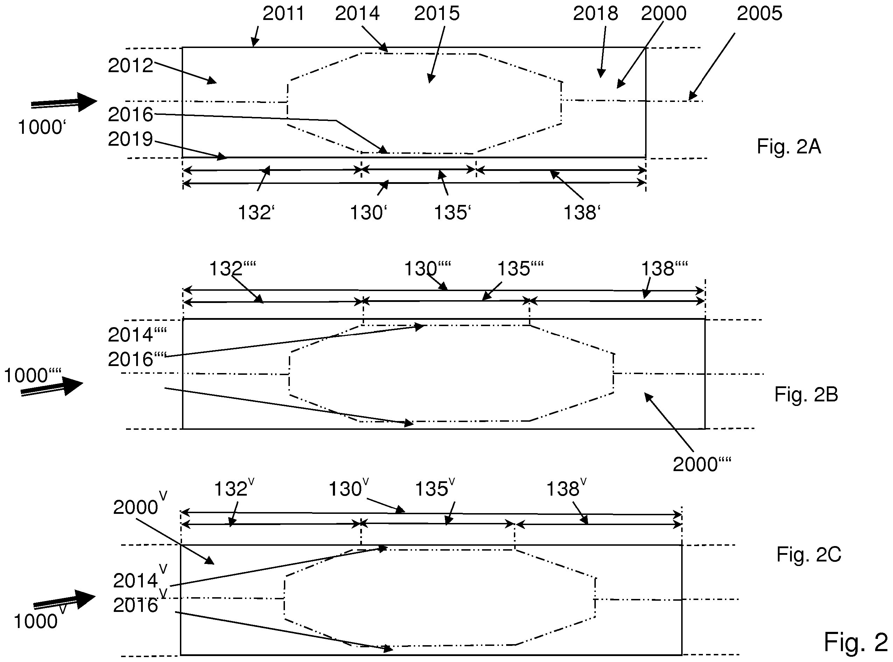

In Fig. 2 A to C such a shape is indicated as a precursor 2000. Therein, a precursor material 2000 for an article to be worn on the lower torso of a wearer, such as a disposable absorbent article such as a disposable diaper or pant is depicted. The affix ', ", "' etc. as added to numerals indicate that the features correspond to the process stages as indicated accordingly in Fig. 1, numerals without affix describe the feature in general. The article comprises a front region and a rear region to be positioned in the front and rear waist region of a wearer during its intended use and a crotch region longitudinally positioned there between. Optionally, the article may comprise absorbent member(s) as well known by a person skilled in the art. An essentially inelastic centre piece may extend from the front waist region of the article through the crotch region to the rear region of the article, exhibiting two lateral side margins. Correspondingly, the precursor 2000 as depicted exhibits front (2012), rear (2018) and crotch (2015) regions aligned along a longitudinally extending centreline 2005. In combination with other materials or webs, the front and rear portions may serve as side panels in the article, such as when these are separated along the longitudinal centreline 2005, and the separated portions are outwardly folded away from the centreline. In order to position the article on a wearer, closure elements may be added, such as melt fusion bonded side seams, adhesively bonded side seams, adhesive closure tapes, and mechanically engaging closure tabs. In the crotch region 2015, most of the material maybe cut out, e.g. along the dash-dotted demarcation lines, such as being discarded or preferably being combined with other materials in the construction of the article. Remaining material stripes 2014 and 2016 connect the front and rear regions in the proximity of the longitudinally extending side margins 2011 and 2019, coinciding with the side margins of the centre strip, respectively, and may serve in the article as leg hoops encircling the legs of a wearer. Thus, in the crotch region the web has an effective material width, in the figure represented by the sum of the widths of regions 2014 and 2016, which is less than the effective material width in the front and rear. The transition between the regions as well as the shape of the regions are only exemplarily indicated, but the form and shape may be chosen over a wide range according to the specific requirements of the article. In general terms, such articles are described in more detail in WO2011/064275. As indicated by the dashed lines in Fig 2, a series of such precursors 2000 may form a web as may be the infeed web 1000' according to the present invention and be a composite made of a nonwoven material and an elastic film or elastic nonwoven web.

The web travels along a web path 1010 around a first roll 110 and a second roll 120. Prior to the first roll 110, the web is indicated as 1000' exhibiting an initial overall pitch length 130', comprising a front region pitch length 132', a rear region pitch length 138' and a crotch region pitch length 135'. It should be noted that for ease of presentation the transition zones are considered to belong to the front or rear regions, respectively. The web leaves the first roll 110 at the first roll tangent point 115 and travels along the path 1010 as web 1000" to the tangent point 125 of the second roll 120, from which the web stretching unit is left with outfeed web 1000"" and corresponding overall outfeed pitch length 130"", which is larger than the original infeed pitch length 130'. The web 1000 is preferably connected to the centre strip in an extended state, such that it retains a certain extensibility during use of the article.

Whilst is it preferred to operate the process such that the two web stripes are simultaneously formed from a single precursor web, the skilled person will readily realize that the two stripes may be formed and treated when starting from two separate web materials.

The stretching is actually occurring between the first (110) and the second (120) roll.

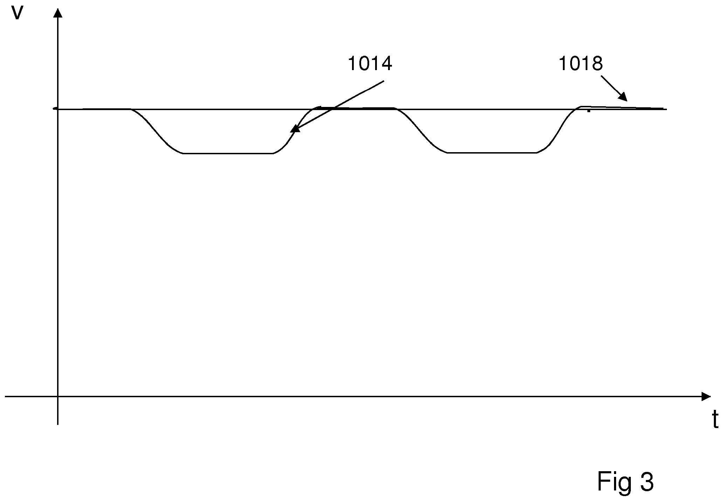

In a first operating mode, as may be explained by referring exemplarily to Fig. 1A the first roll 110 is operated at an oscillating speed v 1014, as indicted in Fig. 3 over time t versus a constant outfeed speed v 1018. In this set up, the speed of the first roll 1014 will typically be lower than the outfeed speed 1018, though for short periods it may also exceed the outfeed speed 1018, as long as the area under the speed curves is smaller, i.e. as long as the integral of the speed profile over time for the speed 1014 of the first roll is smaller than the integral over time of the outfeed speed profile 1018.

In this operating mode, the pitch length may be significantly larger than the length of the stretch gap 140 (i.e. the distance between the tangent points 115 and 125).

With an appropriately chosen surface of the rolls 110 and 120, and the speed differential profile adequately set, the wider sections of the web, e.g. front and rear section 2012 and 2018, will essentially not deform and the pitch length 132'and 132"" as well as 138'and 138"" will be essentially the same, whilst the narrower section, e.g. crotch section 2015, will extend in the stretch zone 140 due to the narrower effective material width being exposed to essentially the same tension force to a pitch 135"" being larger than initial pitch135'. When the web tension is released, the web respectively the precursor or its pieces will retract, and as indicated in Fig. 2C, the overall pitch length will be reduced to a length 130^{v}, as will be lower than 130"" but typically larger the 130' due to the permanent set of the web material. In such a setup it is preferred that the shape of the precursor is symmetric to the longitudinal centre line. Further the overall stretch or pitch increase should preferably be less than 20%, either by selecting a short extension zone (e.g. crotch zone 2014 relative to front and rear zones 2012 and 2019) and/or by imparting only low stress.

Thus, in spite of the varying speed of first roll 110, the outfeed roll 120 can run at constant speed, because the difference in material feed length is compensated by the extension of the web. For this execution, the infeed speed of the web may vary according to the speed changes of the first roll 110. For small extensions, this may be compensated by the web supply. Preferably, a buffering step, such as by using a buffer system (1100*), such as dancer systems, eccentric system, or a servo drive roll system, as all well known in the art, may be implemented in the web path prior to the first roll.

It should be noted, that the above has been described such that the terms "first roll" and "second" correspond to the positioning of the rolls along the web path and then the constant second roll speed corresponds to the outfeed speed. It is also within the scope of the present invention that this order is changed, i.e. the first roll is operated at a constant speed and second roll may have either constant at a speed lower than the first roll speed, or that is oscillating between a speed lower than the first roll speed and a speed equal to the first roll speed or between a lower speed and a higher speed, such that the integral over time of the speed profile of the second roll over a time increment corresponding to a pitch length is smaller than the integral over time of the speed profile of the first roll. In this option, an optional buffering step in a buffer system (1100**) is preferably positioned downstream of the second roll.

It should be noted that the present explanation neglects for simplicity that the transition zones will gradually over the increasing effective width pick up some of the forces and stretch to a certain extent.

In a second operating mode (refer to Fig. 1B), both the first (110) and the second roll (120) can be operated at constant speed, with the speed of the second roll being higher than the one of the first roll, thereby inducing tension in the stress zone 140. For this execution, the pitch length of the article should be less than or close to the stretch zone length, preferably less than 95%, or even less than 80% thereof. Further, it is preferred that the homogeneity of the material is relatively high so as to avoid local overstressing of the material.

It should be noted, that the above has been described such that the terms "first roll" and "second" correspond to the positioning of the rolls along the web path and then the constant second roll speed corresponds to the outfeed speed. It is also within the scope of the present invention that this order is changed, i.e. the first roll is operated at a constant speed and second roll may have either constant at a speed lower than the first roll speed, or that is oscillating between a speed lower than the first roll speed and a speed equal to the first roll speed or between a lower speed and a higher speed, such that the integral over time of the speed profile of the second roll over a time increment corresponding to a pitch length is smaller than the integral over time of the speed profile of the first roll. In this option, an optional buffering step in a buffer system (1100**) is preferably positioned downstream of the second roll.

It should be noted, that equivalent embodiments such as replacing the rolls by other elements, such as drive belt structures, or inserting additional guide means will not depart from the present invention.

## Claims

1. A method for selectively machine directionally stretching a predetermined section of a pre-combined web material, said method comprising the steps of
a) providing an essentially continuous pre-combined web exhibiting an x-direction aligned with the machine direction, a width corresponding to the cross machine direction and a thickness perpendicular thereto at an infeed speed
said web comprising
a1) at least one essentially non-elastic fibrous sub-layer and
a2) at least one elastic sub-layer,
said sub-layers being connected to each other
and said web comprising in a repeating sequence a first region exhibiting a narrower effective material width than a second region, wherein said first region defines a first pitch length, and said second region defines a second pitch length, both defining, optionally with further repeating regions or transition regions, a repeating overall infeed pitch length;
b) providing a selective stretching tool comprising a first and a second roll, defining a stretching zone along the web path between them;
c*) operating said second roll at a preset and essentially constant second roll speed which corresponds essentially to an outfeed speed;
d*) operating said first roll at a predetermined first roll speed, which is
d1*) either constant at a speed lower than said second roll speed
or d2*) oscillating between
d2i*) a speed lower than said second roll speed and a speed equal to said second roll speed
or d2ii*) a lower speed and a higher speed, such that the integral over time of the speed profile of the first roll over a time increment corresponding to a pitch length is smaller than the integral over time of the speed profile of the second roll, whereby said speed differential between said first and said second roll extends said first region of said web to a greater extent than said second region, thereby increasing the first region pitch length as well as the overall pitch length for the outfeed compared to the infeed.

2. A method for selectively machine directionally stretching a predetermined section of a pre-combined web material, said method comprising the steps of
a) providing an essentially continuous pre-combined web exhibiting an x-direction aligned with the machine direction, a width corresponding to the cross machine direction and a thickness perpendicular thereto at an infeed speed
said web comprising
a1) at least one essentially non-elastic fibrous sub-layer and
a2) at least one elastic sub-layer,
said sub-layers being connected to each other
and said web comprising in a repeating sequence a first region exhibiting a narrower effective material width than a second region, wherein said first region defines a first pitch length, and said second region defines a second pitch length, both defining, optionally with further repeating regions or transition regions, a repeating overall infeed pitch length;
b) providing a selective stretching tool comprising a first and a second roll, defining a stretching zone along the web path between them;
c**) operating said first roll at a preset and essentially constant first roll speed which essentially corresponds to the infeed speed of the web;
d**) operating said second roll at a predetermined second roll speed, which is
d1**) either constant at a speed lower than said first roll speed
or d2**) oscillating between
d2i**) a speed higher than said first roll speed and a speed equal to said first roll speed
or d2ii**) a lower speed and a higher speed, such that the integral over time of the speed profile of the second roll over a time increment corresponding to a pitch length is smaller than the integral over time of the speed profile of the first roll, whereby said speed differential between said first and said second roll extends said first region of said web to a greater extent than said second region, thereby increasing the first region pitch length as well as the overall pitch length for the outfeed compared to the infeed.

3. A method according to claim 1 or 2, wherein the outfeed overall pitch length exceeds the infeed overall pitch length by less than 20%, preferably less than 10% but more than 3%, preferably 5%, based on the infeed overall pitch length.

4. A method according to claim 1 or 2, wherein said first pitch length is increased from the infeed to the outfeed by less than 50%, preferably less than 30%, more preferably less than 20%, based on the first infeed pitch length.

5. A method according to any of claims 1 or 2, wherein step d*) or d**), respectively is executed at a first roll speed, which is constant at a speed lower than the outfeed speed, wherein said pitch length after stretching is less than 100% of the stretch zone.

6. A method according to any of claims 1 or 2, wherein step d*) or d**) is executed at a first roll speed, which is oscillating between a lower speed and a higher speed, such that the integral over time of the speed profile of the first roll over a time increment corresponding to a pitch length is smaller than the integral over time of the speed profile of the second roll, and wherein said stretch zone length is less than 50%, preferably less than 20% of the infeed pitch length, based on the infeed pitch length.

7. A method of forming a disposable article,
said article comprising a front portion and a rear region connected by a crotch region, thereby defining the length direction of the article,
said article further comprising a centre strip positioned in said front, crotch, and rear portion,
said method of forming said article comprising the steps of
- positioning an essentially inelastic web material for forming the centre strip of said article such that the machine direction of the method is parallel to the length direction of the article, such that said inelastic web material exhibits two longitudinally extending margins,
- selectively machine directionally stretching at least one predetermined section of a pre-combined web material according to any of the preceding claims;
- creating two web stripes by separating a selectively machine directionally stretching at least one a predetermined section of a pre-combined web material along an essentially longitudinally oriented separation line;
- positioning and connecting a first and a second of said selectively stretched materials adjacently to the lateral margins of said centre strip whilst said stretched material are in a stretched state;
- adding absorbent member;
- adding side closure elements preferably selected from the group consisting of melt fusion bonded side seams, adhesively bonded side seams, adhesive closure tapes

## Patentansprüche

1. Verfahren zum selektiven Strecken eines vorbestimmten Abschnitts eines vorkombinierten Bahnmaterials in Maschinenrichtung, wobei das Verfahren die Schritte umfasst
a) Bereitstellen einer im Wesentlichen kontinuierlichen vorkombinierten Bahn, die eine mit der Maschinenrichtung ausgerichtete x-Richtung, eine der Maschinenquerrichtung entsprechende Breite und eine dazu senkrechte Dicke bei einer Einlaufgeschwindigkeit aufweist,
wobei die Bahn umfasst
a1) mindestens eine im Wesentlichen nicht elastische faserige Teilschicht und
a2) mindestens eine elastische Teilschicht,
wobei die Teilschichten miteinander verbunden sind
und wobei die Bahn in einer sich wiederholenden Sequenz einen ersten Bereich umfasst, der eine schmalere effektive Materialbreite als ein zweiter Bereich aufweist, wobei der erste Bereich eine erste Teilungslänge definiert und der zweite Bereich eine zweite Teilungslänge definiert, die beide, gegebenenfalls mit weiteren Wiederholungs- oder Übergangsbereichen, eine sich wiederholende Gesamtteilungslänge des Einlaufs definieren;
b) Bereitstellen eines selektiven Streckwerkzeugs, umfassend eine erste und eine zweite Walze, die eine Streckzone entlang des Bahnpfades zwischen ihnen definieren;
c *) Betreiben der zweiten Walze mit einer voreingestellten und im Wesentlichen konstanten Geschwindigkeit der zweiten Walze, die im Wesentlichen einer Auslaufgeschwindigkeit entspricht;
d *) Betreiben der ersten Walze mit einer vorbestimmten Geschwindigkeit der ersten Walze, welche
d1 *) entweder konstant bei einer Geschwindigkeit, die niedriger als die Geschwindigkeit der zweiten Walze ist
oder d2 *) oszillierend zwischen
d2i *) einer Geschwindigkeit, die niedriger als die Geschwindigkeit der zweiten Walze ist, und einer Geschwindigkeit, die der Geschwindigkeit der zweiten Walze entspricht
oder d2ii *) einer niedrigeren Geschwindigkeit und einer höheren Geschwindigkeit, so dass das Zeitintegral des Geschwindigkeitsprofils der ersten Walze über ein Zeitinkrement, das einer Teilungslänge entspricht, kleiner ist als das Zeitintegral des Geschwindigkeitsprofils der zweiten Walze,
wobei die Geschwindigkeitsdifferenz zwischen der ersten und der zweiten Walze den ersten Bereich der Bahn stärker ausdehnt als den zweiten Bereich,
wodurch die Teilungslänge des ersten Bereichs sowie die Gesamtteilungslänge für den Auslauf im Vergleich zum Einlauf erhöht werden.

2. Verfahren zum selektiven Strecken eines vorbestimmten Abschnitts eines vorkombinierten Bahnmaterials in Maschinenrichtung, wobei das Verfahren die Schritte umfasst
a) Bereitstellen einer im Wesentlichen kontinuierlichen vorkombinierten Bahn, die eine mit der Maschinenrichtung ausgerichtete x-Richtung, eine der Querrichtung der Maschine entsprechende Breite und eine dazu senkrechte Dicke bei einer Einlaufgeschwindigkeit aufweist,
wobei die Bahn umfasst
a1) mindestens eine im Wesentlichen nicht elastische faserige Teilschicht und
a2) mindestens eine elastische Teilschicht,
wobei die Teilschichten miteinander verbunden sind
und wobei die Bahn in einer sich wiederholenden Sequenz einen ersten Bereich umfasst, der eine schmalere effektive Materialbreite als ein zweiter Bereich aufweist, wobei der erste Bereich eine erste Teilungslänge definiert und der zweite Bereich eine zweite Teilungslänge definiert, die beide, gegebenenfalls mit weiteren Wiederholungs- oder Übergangsbereichen, eine sich wiederholende Gesamtteilungslänge des Einlaufs definieren;
b) Bereitstellen eines selektiven Streckwerkzeugs, umfassend eine erste und eine zweite Walze, die eine Streckzone entlang des Bahnpfades zwischen ihnen definieren;
c **) Betreiben der ersten Walze mit einer voreingestellten und im Wesentlichen konstanten Geschwindigkeit der ersten Walze, die im Wesentlichen der Einlaufgeschwindigkeit der Bahn entspricht;
d **) Betreiben der zweiten Walze mit einer vorbestimmten Geschwindigkeit der zweiten Walze, welche
d1 **) entweder konstant bei einer Geschwindigkeit, die niedriger als die der ersten Walze ist
oder d2 **) oszillierend zwischen
d2i **) einer höheren Geschwindigkeit als die Geschwindigkeit der ersten Walze und einer Geschwindigkeit, die der der ersten Walze entspricht
oder d2ii **) einer niedrigeren Geschwindigkeit und einer höheren Geschwindigkeit, so dass das Zeitintegral des Geschwindigkeitsprofils der zweiten Walze über ein Zeitinkrement, das einer Teilungslänge entspricht, kleiner ist als das Zeitintegral des Geschwindigkeitsprofils der ersten Walze,
wobei die Geschwindigkeitsdifferenz zwischen der ersten und der zweiten Walze den ersten Bereich der Bahn stärker ausdehnt als den zweiten Bereich,
wodurch die Teilungslänge des ersten Bereichs sowie die Gesamtteilungslänge für den Auslauf im Vergleich zum Einlauf erhöht werden.

3. Verfahren nach Anspruch 1 oder 2, wobei die Gesamtteilungslänge des Auslaufs die Gesamtteilungslänge des Einlaufs um weniger als 20%, vorzugsweise weniger als 10%, aber mehr als 3%, vorzugsweise 5%, basierend auf der Gesamtteilungslänge des Einlaufs, überschreitet.

4. Verfahren nach Anspruch 1 oder 2, wobei die erste Teilungslänge von dem Einlauf bis zum Auslauf um weniger als 50%, vorzugsweise weniger als 30%, noch bevorzugterweise weniger als 20% erhöht wird, basierend auf der ersten Einlaufteilungslänge.

5. Verfahren nach einem der Ansprüche 1 oder 2, wobei der Schritt d *) bzw. d **) bei einer ersten Walzengeschwindigkeit ausgeführt wird, die konstant bei einer Geschwindigkeit ist, die niedriger als die Auslaufgeschwindigkeit ist, wobei die Teilungslänge nach dem Strecken weniger als 100% der Streckzone beträgt.

6. Verfahren nach einem der Ansprüche 1 oder 2, wobei der Schritt d *) oder d **) bei einer ersten Walzengeschwindigkeit ausgeführt wird, die zwischen einer niedrigeren Geschwindigkeit und einer höheren Geschwindigkeit oszilliert,
so dass das Zeitintegral des Geschwindigkeitsprofils der ersten Walze über ein Zeitinkrement, das einer Teilungslänge entspricht, kleiner ist als das Zeitintegral des Geschwindigkeitsprofils der zweiten Walze, wobei die Streckzonenlänge weniger als 50%, vorzugsweise weniger als 20% der Einlaufteilungslänge beträgt, basierend auf der Einspeisungsteilungslänge.

7. Ein Verfahren zum Formen eines Einwegartikels,
wobei der Artikel einen vorderen Abschnitt und einen hinteren Abschnittumfasst, die durch einen Schrittabschnitt verbunden sind, wodurch die Längsrichtung des Artikels definiert wird;
wobei der Artikel ferner einen Mittelstreifen umfasst, der in dem vorderen, Schritt- und hinteren Abschnitt positioniert ist,
wobei das Verfahren zum Formen des Artikels die Schritte umfasst
- Positionieren eines im wesentlichen unelastischen Bahnmaterials zum Bilden des Mittelstreifens des Artikels, so dass die Maschinenrichtung des Verfahrens parallel zur Längsrichtung des Artikels ist, so dass das unelastische Bahnmaterial zwei sich in Längsrichtung erstreckende Ränder aufweist;
- selektives Strecken in Maschinenrichtung von mindestens einem vorbestimmten Abschnitt eines vorkombinierten Bahnmaterials gemäß einem der vorhergehenden Ansprüche;
- Erzeugen von zwei Bahnstreifen durch Trennen eines vorkombinierten Bahnmaterials mit mindestens einem selektiv in Maschinenrichtung gedehnten vorbestimmten Abschnitts entlang einer im Wesentlichen in Längsrichtung ausgerichteten Trennlinie;
- Positionieren und Verbinden eines ersten und eines zweiten der selektiv gestreckten Materialien neben den seitlichen Rändern des Mittelstreifens, während sich das gestreckte Material in einem gestreckten Zustand befindet;
- Hinzufügen eines absorbierenden Elements;
- Hinzufügen von Seitenverschlusselementen, die vorzugsweise ausgewählt sind aus der Gruppe bestehend aus schmelzverbundenen Seitennähten, geklebten Seitennähten, klebenden Verschlussbändern und mechanisch verhakende Verschlusslaschen.

## Revendications

1. Procédé pour étirer sélectivement dans le sens de la machine une section prédéterminée d'un matériau de bande pré-combiné, ledit procédé comprenant les étapes de
a) fournir une bande pré-combinée essentiellement continue présentant une direction x alignée avec la direction de la machine, une largeur correspondant à la direction transversale de la machine et une épaisseur perpendiculaire à celles-ci à une vitesse d'entrée ladite bande comprenant
a1) au moins une sous-couche fibreuse essentiellement non élastique et
a2) au moins une sous-couche élastique,
lesdites sous-couches étant connectées les unes aux autres
et ladite bande comprenant dans une séquence de répétition une première région présentant une largeur de matériau efficace plus étroite qu'une seconde région, dans lequel ladite première région définit une première longueur de pas, et ladite seconde région définit une seconde longueur de pas, les deux définissant, éventuellement avec d'autres régions de répétition ou des régions de transition, une longueur de pas d'entrée totale répétée;
b) fournir un outil d'étirage sélectif comprenant un premier et un second rouleau, définissant une zone d'étirage le long du trajet de bande entre eux ;
c *) faire fonctionner ledit deuxième rouleau à une deuxième vitesse de rouleau prédéterminée et essentiellement constante qui correspond essentiellement à une vitesse de sortie ;
d *) faire fonctionner ledit premier rouleau à une première vitesse de rouleau prédéterminée, qui est
d1 *) soit constante à une vitesse inférieure à ladite seconde vitesse de rouleau
ou d2 *) oscillant entre
d2i *) une vitesse inférieure à ladite seconde vitesse de rouleau et une vitesse égale à ladite seconde vitesse de rouleau
ou d2ii *) une vitesse plus faible et une vitesse plus élevée, telles que l'intégrale du temps du profil de vitesse du premier rouleau, sur un incrément de temps correspondant à une longueur de pas, est inférieure à l'intégrale du temps du profil de vitesse du deuxième rouleau,
moyennant quoi ledit différentiel de vitesse entre ledit premier et ledit deuxième rouleau étend ladite première région de ladite bande dans une plus grande mesure que ladite deuxième région, augmentant ainsi la longueur de pas de la première région ainsi que la longueur de pas totale pour la sortie par rapport à l'entrée.

2. Procédé pour étirer sélectivement dans le sens de la machine une section prédéterminée d'un matériau de bande pré-combiné, ledit procédé comprenant les étapes de
a) fournir une bande pré-combinée essentiellement continue présentant une direction x alignée avec la direction de la machine, une largeur correspondant à la direction transversale de la machine et une épaisseur perpendiculaire à celles-ci à une vitesse d'entrée,
ladite bande comprenant
a1) au moins une sous-couche fibreuse essentiellement non élastique et
a2) au moins une sous-couche élastique,
lesdites sous-couches étant connectées les unes aux autres
et ladite bande comprenant dans une séquence de répétition une première région présentant une largeur de matériau efficace plus étroite qu'une seconde région, dans lequel ladite première région définit une première longueur de pas, et ladite seconde région définit une seconde longueur de pas, les deux définissant, éventuellement avec d'autres régions de répétition ou des régions de transition, une longueur de pas d'totale répétée;
b) fournir un outil d'étirage sélectif comprenant un premier et un second rouleau, définissant une zone d'étirage le long du trajet de bande entre eux ;
c **) faire fonctionner ledit premier rouleau à une vitesse de premier rouleau prédéterminée et essentiellement constante qui correspond essentiellement à la vitesse d'entrée de la bande;
d **) faire fonctionner ledit deuxième rouleau à une deuxième vitesse de rouleau prédéterminée, qui est
d1 **) soit constante à une vitesse inférieure à ladite première vitesse de rouleau
ou d2 **) oscillant entre
d2i **) une vitesse supérieure à ladite première vitesse de rouleau et une vitesse égale à ladite première vitesse de rouleau
ou d2ii **) une vitesse inférieure et une vitesse plus élevée, telles que l'intégrale du temps du profil de vitesse du deuxième rouleau sur un incrément de temps correspondant à une longueur de pas, est inférieure à l'intégrale du temps du profil de vitesse du premier rouleau,
moyennant quoi ledit différentiel de vitesse entre ledit premier et ledit deuxième rouleau étend ladite première région de ladite bande dans une plus grande mesure que ladite deuxième région, augmentant ainsi la longueur de pas de la première région ainsi que la longueur de pas totale pour la sortie par rapport à l'entrée.

3. Procédé selon la revendication 1 ou 2, dans lequel la longueur de pas totale de sortie dépasse la longueur de pas totale d'entrée de moins de 20%, de préférence moins de 10% mais plus de 3%, de préférence 5%, sur la base de la longueur de pas totale d'entrée.

4. Procédé selon la revendication 1 ou 2, dans lequel ladite première longueur de pas est augmentée de l'entrée à la sortie de moins de 50%, de préférence de moins de 30%, mieux encore de moins de 20%, basé sur la première longueur de pas d'entrée.

5. Procédé selon l'une quelconque des revendications 1 ou 2, dans lequel l'étape d *) ou d **), respectivement, est exécutée à une première vitesse de rouleau, qui est constante à une vitesse inférieure à la vitesse de sortie, dans lequel ladite longueur de pas après étirement est moins de 100% de la zone d'étirement.

6. Procédé selon l'une quelconque des revendications 1 ou 2, dans lequel l'étape d *) ou
d **) est exécutée à une première vitesse de rouleau, qui oscille entre une vitesse inférieure et une vitesse plus élevée, de telle sorte que l'intégrale du temps du profil de vitesse du premier rouleau, sur un incrément de temps correspondant à une longueur de pas, est plus petit que l'intégrale du temps du profil de vitesse du second rouleau,
et dans lequel ladite longueur de zone d'étirement est inférieure à 50%, de préférence inférieure à 20% de la longueur de pas d'entrée, basée sur la longueur de pas d'entrée.

7. Procédé de formation d'un article jetable,
ledit article comprenant une partie avant et une région arrière reliées par une région d'entrejambe, définissant ainsi la direction de la longueur de l'article,
ledit article comprenant en outre une bande centrale positionnée dans lesdites parties avant, entrejambe et arrière,
ledit procédé de formation dudit article comprenant les étapes de
- positionner un matériau de bande essentiellement inélastique pour former la bande centrale dudit article de telle sorte que le sens machine du procédé soit parallèle au sens de la longueur de l'article, de sorte que ledit matériau en bande non élastique présente deux marges s'étendant longitudinalement,
- étirer sélectivement dans le sens de la machine au moins une section prédéterminée d'un matériau de bande pré-combiné selon l'une quelconque des revendications précédentes;
- création de deux bandes en séparant un étirage sélectif dans le sens de la machine d'au moins une section prédéterminée d'un matériau de bande pré-combiné le long d'une ligne de séparation essentiellement orientée longitudinalement ;
- positionner et connecter un premier et un second desdits matériaux étirés sélectivement de manière adjacente aux marges latérales de ladite bande centrale tandis que ledit matériau étiré est dans un état étiré ;
- ajouter un élément absorbant ;
- ajouter d'éléments de fermeture latéraux de préférence choisis dans le groupe constitué par des coutures latérales liées par fusion, des coutures latérales liées par adhésif, des bandes de fermeture adhésives, des pattes de fermeture engageant mécaniquement.
